(19) Europäisches Patentamt
European Patent Office
Office européen des brevets

(11) EP 3 471 692 B1

(12) **EUROPEAN PATENT SPECIFICATION**

(45) Date of publication and mention of the grant of the patent:
**30.10.2019 Bulletin 2019/44**

(21) Application number: **17722833.5**

(22) Date of filing: **16.05.2017**

(51) Int Cl.:
***A61K 8/34*** *(2006.01)* ***A61K 8/49*** *(2006.01)*
***A61K 8/67*** *(2006.01)* ***A61Q 19/02*** *(2006.01)*
***A61Q 19/08*** *(2006.01)*

(86) International application number:
**PCT/EP2017/061676**

(87) International publication number:
**WO 2017/215863 (21.12.2017 Gazette 2017/51)**

(54) **METHOD AND COSMETIC COMPOSITION FOR ENHANCED TRANSDERMAL DELIVERY OF ALKYL SUBSTITUTED RESORCINOL**

VERFAHREN UND KOSMETISCHE ZUSAMMENSETZUNG ZUR VERBESSERTEN TRANSDERMALEN VERABREICHUNG VON RESORCINOLEN

PROCÉDÉ ET COMPOSITION COSMÉTIQUE POUR ADMINISTRATION TRANSDERMIQUE AMÉLIORÉE DE RÉSORCINOLS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.06.2016 EP 16174517**

(43) Date of publication of application:
**24.04.2019 Bulletin 2019/17**

(73) Proprietors:
- **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**
- **Unilever PLC**
**London, Greater London EC4Y 0DY (GB)**
Designated Contracting States:
**CY GB IE MT**

(72) Inventors:
- **DAMODARAN, Anita**
**Whitefield**
**Bangalore 560 066 (IN)**
- **JOSHI, Manoj, Kumar**
**Whitefield**
**Bangalore 560 066 (IN)**
- **KUNJUPILLAI, Balu**
**Whitefield**
**Bangalore 560 066 (IN)**
- **SHARIFF, Rezwan**
**Whitefield**
**Bangalore 560 066 (IN)**

(74) Representative: **Corsten, Michael Allan**
**Unilever N.V.**
**Unilever Patent Group**
**Olivier van Noortlaan 120**
**3133 AT Vlaardingen (NL)**

(56) References cited:
**WO-A1-2015/059001** **WO-A2-2013/030794**
**US-A- 5 411 741**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 471 692 B1

## Description

### Field of the invention

The present invention relates to method and cosmetic composition for enhanced transdermal delivery (TDD) of alkyl substituted resorcinol.

### Background of the invention

It is a universal desire to look younger and have healthy, even-toned complexion. The indicators of healthy skin are uniformity of skin colour and texture. Age spots, melasma, acne lesions and other forms of hyperpigmentation is an unwelcome change in the characteristics of skin, especially facial skin. In at least some parts of the world, people desire lighter or fairer skin. In some other parts, lighter skin may not be a desire, but an even-toned skin certainly is. Accordingly, there is need for cosmetic compositions that meet the expectations of consumers.

Several attempts in the past led to the development of a variety of skin care compositions and regimen. However, at least some active ingredients exhibit efficacy or are associated with side effects, such as toxicity and irritation. Therefore, there is continuing need for newer ingredients and more potent products. In addition, the need exists for newer ways and means to enhance the efficacy of known ingredients.

At various stages of our life, human beings get concerned about excessive pigmentation of skin and it often leads to the desire to eliminate it or at least reduce it to some extent.

Melanogenesis leads to skin pigmentation. It involves the following stages:

Tyrosine → DOPA → Dopaquinone → Dopachrome → Melanin

The first two reactions are catalysed by tyrosinase. The activity of tyrosinase is promoted by the action of a melanocyte-stimulating hormone or by UV rays. It is known that a substance could cause depigmentation if it acts directly on the vitality of the epidermal melanocytes where melanogenesis normally occurs and/or if it interferes with one of the stages in melanin biosynthesis. In view of the role played by tyrosinase, assays linked to its inhibition are often used to screen potential skin lightening agents.

European Patent Application EP341664 A1 and PCT International Publication WO 99/15148 A1 refer to certain resorcinol derivatives as tyrosinase inhibitors.

Resorcinol (1,3-benzenediol) derivatives have been used for cosmetic benefits to skin and hair. 4-Substituted resorcinol derivatives are used for skin lightening; see, for example, U.S. Pat. No. 4,959,393, U.S. Pat. No. 6,132,740, U.S. Pat. No. 6,504,037, U.S. Patent Application Publication No. 2008/0131382, and Japanese Published Patent Application Nos. JP 2001-01 0925 and JP 2000-327557. Resorcinol derivative dimers, which are inhibitors of tyrosinase, are disclosed in U.S. Pat. No. 5,399,785. Resorcinol-type skin lightening agents, which could be synthesized using coumarin as starting material, are disclosed in U.S. Patent Application Publication No. 2004/0042983. Certain resorcinol derivatives are widely used in cosmetic compositions. In particular, 4-substituted alkyl resorcinols are useful in cosmetic compositions for skin lightening and the derivatives used more widely are 4-hexyl, 4-isopropyl, 4-butyl, 4-ethyl I and 4-phenylethyl resorcinol.

US2014256830A (Unilever) discloses compositions that have resorcinol derivatives for the use of treating, regulating or preventing a skin condition characterized by oxidative stress or degenerative processes. Methods of preventing, lightening or reducing the appearance of visible discontinuities of the skin resulting from skin pigmentation, skin aging or other disorders are also disclosed therein.

US2013281507A, EP0623339 A1 and WO13190483 A1 (All by L'Oreal) discloses certain derivatives of resorcinol for depigmenting, lightening and/or whitening keratin materials.

While human skin provides an ideal site for administration of active ingredients intended to perform one or more defined functions, it presents some amount of resistance to the permeation of most compounds. The underlying reasons therefor and the mechanisms to aid or increase permeation is studied under the topic of TDD.

Most TDD compositions, (e.g., therapeutic or cosmetic compositions) achieve epidermal penetration by using a skin penetration enhancing carrier or vehicle. Such carrier or vehicles (which are compounds or mixtures of compounds) are known in the art as "penetration enhancers'" or "skin enhancers." While some skin enhancers in the literature enhance transdermal absorption, they possess certain drawbacks, which include toxicity, irritation and thinning effect.

An alternative approach is to locally damage the skin via micropunctures or abrasions to facilitate penetration of active ingredients but such a method is not suited for all skin types and age groups.

Cosmetic compositions containing resorcinol derivatives are usually applied once or twice daily. Upon application of the composition, the actives therein, including resorcinol derivatives, need to reach their site of action by manoeuvring through various layers of skin (transdermal delivery). Using the commonly known methods, more amount could be delivered by iontophoresis, which is a method of aiding the passage of polar molecules into the skin by applying a small electrical current or alternatively by a skin penetration enhancer (SPE) disclosed for example, in Tropical Journal

of Pharmaceutical Research, April 2009; 8 (2): 173-179 (Inayat Bashir Pathan and C. Mallikarjuna Setty). Examples include sulphoxides, azone, pyrrolidones, fatty acid, essential oils, terpenes and terpenoids.

In yet another method, US2002120225 A (McDaniel) discloses a method for enhancing the transport of an active agent through mammalian skin in which the skin is exposed to ultrasound after applying an active agent to the skin, wherein the step of applying an active agent to the skin comprises injecting the active agent into the skin. The active agent comprises one of several ingredients listed therein.

In yet another method, US5411741 (Nardo Zaias) discloses encapsulating an effective amount of melanin inhibiting compound such as of 3-pyridinecarboxamide (niacinamide) with liposome, suspending the encapsulated compound within a topical vehicle and topically applying to the epidermis of the skin the suspended and encapsulated melanin inhibiting compound whereby liposomes are transdermally delivered to the basal cell region where it interferes with the biochemical synthesis of melanin in situ that results in depigmentation of the skin.

WO2013/030794 A2 (Kasraee Behrooz) discloses a skin depigmentation composition containing picolinamide or isonicotinamideand said composition further comprises 4-substituted resorcinol.

WO2015/059001 (Unilever) discloses skin lightening composition comprising a pyridine compound defined by claim1 therein and a dermatologically acceptable base. A skin lightening composition disclosed therein further comprises an additional skin lightening agents including 4-ethyl resorcinol, 4-hexyl resorcinol and phenylethyl resorcinol.

Methods known thus far for TDD of actives beneficial to the skin like e.g. actives that deliver skin lightening, have some or the other drawbacks associated with them or involve additional steps of encapsulation as described above. Therefore, there is a need to deliver enhanced TDD of such actives using a way that overcomes all or at least some of the drawbacks associated with methods disclosed thus far.

We have surprisingly determined that picolinamide as well as isonicotinamide, independently, aid the TDD of at least some alkyl substituted resorcinol when picolinamide and an alkyl substituted resorcinol or isonicotinamide and an alkyl substituted resorcinol are present together in a cosmetic composition.

This observation provides an industrially applicable mode for cosmetic compositions, such as creams and lotions containing alkyl substituted resorcinol and at least one of picolinamide or isonicotinamide.

In view of enhanced TDD of the alkyl substituted resorcinol, the invention provides an effective method to formulate compositions, which could contain significantly lower amount of alkyl substituted resorcinol. Alternatively, the invention provides an effective method to formulate compositions, which are faster- or longer-acting as compared to conventional formulations.

## Summary of the invention

In accordance with a first aspect is disclosed a cosmetic composition comprising:

(i) at least one pyridine compound of Formula I or II:

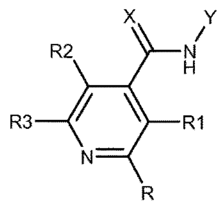

Formula I

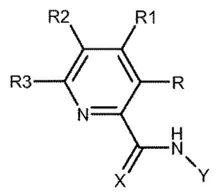

Formula II

where, in said Formula I and in said Formula II, X is S or O; R, $R_1$, $R_2$, $R_3$ are either -H, -SH, -OH or a $C_{1-18}$ saturated or unsaturated, linear, branched or cyclic hydrocarbon group; Y is -H, -SH or $C_{1-18}$ saturated or unsaturated, linear, branched or cyclic hydrocarbon group;

(ii) an alkyl substituted resorcinol; and,

(iii) a dermatologically acceptable base .

In accordance with a second aspect is disclosed a method for enhancing transdermal delivery of an alkyl substituted resorcinol, comprising the steps of:

(i) providing a cosmetic composition in accordance with the first aspect; and,

(ii) applying an effective amount of said composition to skin.

In accordance with a third aspect is disclosed use of a pyridine compound of Formula I or II for enhancing TDD

of an alkyl substituted resorcinol.

In accordance with a fourth aspect is disclosed use of a pyridine compound of Formula I or II in the preparation of a cosmetic composition for enhancing TDD of an alkyl substituted resorcinol.

In accordance with a fifth aspect is disclosed a composition for use to enhance TDD of an alkyl substituted resorcinol, comprising:

(i) at least one pyridine compound of Formula I or II:

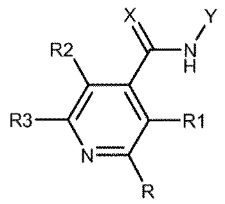
Formula I

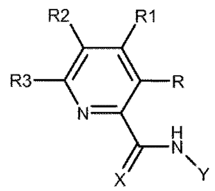
Formula II

where, in said Formula I and in said Formula II, X is S or O; R, $R_1$, $R_2$, $R_3$ are either -H, -SH, -OH or a $C_{1-18}$ saturated or unsaturated, linear, branched or cyclic hydrocarbon group; Y is -H, -SH or $C_{1-18}$ saturated or unsaturated, linear, branched or cyclic hydrocarbon group;
(ii) an alkyl substituted resorcinol; and,
(iii) a dermatologically acceptable base.

In accordance with a sixth aspect is disclosed a method of lightening skin comprising a step of applying thereon a composition in accordance with the first aspect.

**Detailed description of the invention**

Definitions

As used herein, a "subject" is a mammal, particularly a human. Use "in" a subject comprises use "on" a subject that is external use according to the context of the use.

As used herein, the terms "even-toning", "whitening", "lightening" and "depigmentation" agent are used interchangeably throughout this document. For purposes of skin lightening, topical application of skin lightening agent should have a lightening effect on only the area to be treated, preferably produce no or minimal irritation, preferably produce no or minimal post-inflammatory secondary pigmentation, and preferably not cause an allergic reaction. In addition, the skin lightening should be effective for normal cutaneous pigmentation and its excesses, including, but not limited to, lentigo senilis, chloasma, cicatrical brown spots, and hyperpigmentation after use of photosensitizing products. Preferably, the skin lightening should be effective while simultaneously providing anti-aging skin benefits.

A used herein, a "skin-lightening or pigmentation reducing amount of a compounds means an amount or concentration of the compound capable of detectably lightening skin or reducing pigmentation in a subject, such as a human, as determined by any standard assay. The active compound is tadministered in a dermatological or pharmaceutical composition for a standard course of treatment that produces the desired result of skin depigmentation.

As used herein, "administering to skin in need of such treatment" means contacting (e.g., by use of the hands or an applicator such as, but not limited to, a wipe, tube, roller, spray, or patch) the area of skin in need of such treatment or an area of skin proximate to the area of skin in need of such treatment.

As used herein, "composition" means a composition suitable for topical administration to the skin.

As used herein, the term "cosmetics" includes make-up, foundation, and skin care products. The term "make-up" refers to products that leave color on the face, including foundations. The term "foundation" refers to liquid, creme, mousse, pancake, compact, concealer, or like products that even out the overall coloring of the skin. Foundation is manufactured to work better over moisturized and/or oiled skin. The term "skin care products" refers to products used to treat or otherwise care for, moisturize, improve, or clean the skin. Products contemplated by the phrase "skin care products" include, but are not limited to, adhesives, bandages, anhydrous occlusive moisturizers, antiperspirants, facial wash cleaners, cold cream, deodorants, soaps, powders, tissues, wipes and solid emulsion compact.

As used herein, the term "cosmetically-acceptable" or "dermatologically-acceptable" means that the compositions or components thereof so-described are suitable for use in contact with skin, particularly human skin, without undue toxicity, incompatibility, instability, irritation, or allergic response.

As used herein, the term "cosmetically acceptable carrier", "cosmetically acceptable excipient", "dermatologically acceptable carrier" or "dermatologically acceptable excipient" includes any and all solvents, dispersion media, coatings, antibacterial and antifungal agents, isotonic and absorption delaying agents and the like, that are cosmetically acceptable or dermatologically acceptable. The use of such media and agents for cosmetically active substances is well known in the art. Except insofar as any conventional media or agent is incompatible with the active ingredient, its use in the cosmetic compositions is contemplated. Supplementary active ingredients can also be incorporated into the compositions. Dermatologically acceptable carriers are suitable for topical application to the skin, have good aesthetic properties, are compatible with the active agents described herein and any other components, and cause minimal or no safety or toxicity concerns. An effective amount of base is from about 50% to about 99.99% or about 50% to about 99%, preferably from about 80% to about 99.9% or about 75% to about 99%, more preferably from about 90% to about 98%, and most preferably from about 90% to about 95% or about 85% to about 95% of the composition. The percentages are % by weight.

As used herein, the term "effective amount" refers to that amount of a compound described herein that is sufficient to effect treatment, as defined below, when administered to a subject in need of such treatment. The effective amount will vary depending upon the subject and skin condition or disease condition being treated and the like, all of which can readily be determined by one of ordinary skill in the art. As used herein, "regulating a skin condition" includes regulating the appearance of a skin condition, including visible discontinuities in skin such as, but not limited to, coloration, discoloration, and unwanted pigmentation. Regulating a skin condition includes even-toning the skin and reducing pigmentation.

As used herein, the term "topical application" means to apply or spread the compositions described herein onto the surface of the skin.

As used herein, the terms "treat" and "treating", and the like refer to reversing, alleviating, or inhibiting the progress of, the disorder or condition to which such term applies, or one or more symptoms of such disorder or condition. The term "treatment", as used herein, refers to the act of treating, as "treating" is defined immediately above. The term "treatment" or "treating" includes the reduction in appearance of skin imperfections irrelevant of the mechanism of action. One of ordinary skill in the art will appreciate that the endpoint of treatment chosen in a particular case will vary according to the disease, condition, or disorder being treated, the outcome desired by the patient, subject, or treating physician, and other factors. Where the composition is being used to lighten skin color such as, for example, to reverse hyperpigmentation caused by, for example, diseases such as melasma or age spots, any one of a number of endpoints can be chosen. For example, endpoints can be defined subjectively such as, for example, when the subject is simply "satisfied" with the results of the treatment. For pharmacological compositions, the endpoint can be determined by the patient's, subject's, or the treating physician's, satisfaction with the results of the treatment. Alternatively, endpoints can be defined objectively. For example, the patient's or subject's skin in the treated area can be compared to a color chart. Treatment is terminated when the color of the skin in the treated area is similar in appearance to a color on the chart. Alternatively, the reflectance of the treated skin can be measured, and treatment can be terminated when the treated skin attains a specified reflectance. Alternatively, the melanin content of the treated skin can be measured. Treatment can be terminated when the melanin content of the treated skin reaches a specified value. Melanin content can be determined in any way known to the art, including by histological methods, with or without enhancement by stains for melanin.

"Skin Care Composition" as used herein, is meant to include a composition for topical application to skin of mammals, especially humans. Such a composition may be generally classified as leave-on or rinse off, and includes any product applied to a human body for improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of skin care compositions include leave-on skin lotions and creams, shower gels, toilet bars, antiperspirants, deodorants, shave creams, depilatories, foundations and sunscreen lotions.

"Skin" as used herein is meant to include skin on the face and body (e.g., neck, chest, back, arms, underarms, hands, legs, buttocks and scalp). The inventive compositions are especially useful for application to skin areas with the highest incidence of hyperpigmentation such as-face and underarms, most preferably the inventive compositions are skin lightening compositions, e.g., creams, deodorants and antiperspirants.

"Lightening" as used herein, means the lightening of skin color as well as the lightening of spots (hyperpigmentation) on the skin, like age spots and freckles.

<u>Cosmetic compositions in accordance with the invention</u>

<u>Pyridine Compound</u>

Cosmetic compositions in accordance with this invention comprise at least one pyridine compound of Formula I or II:

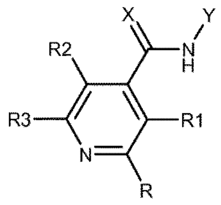

Formula I

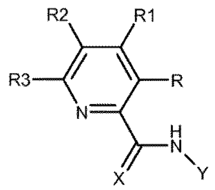

Formula II

where, in said Formula I and in said Formula II, X is S or O; R, $R_1$, $R_2$, $R_3$ are either -H, -SH, -OH or a $C_{1-18}$ saturated or unsaturated, linear, branched or cyclic hydrocarbon group; Y is -H, -SH or $C_{1-18}$ saturated or unsaturated, linear, branched or cyclic hydrocarbon group;

It is preferred that the amount of the pyridine compound of Formula I or II is 0.05 to 8 % by weight of the composition. More preferably it is 0.1 to 5 % by weight of the composition.

It is preferred that the pyridine compound is at least one of Isonicotinamide (Structure-A) or Picolinamide (Structure B). They are examples of Formula I and II respectively.

| 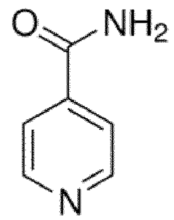 | 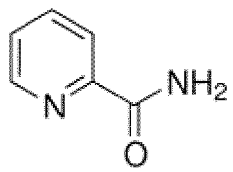 |
|---|---|
| [Structure-A] | [Structure-B] |

It is preferred that compositions in accordance with the invention comprise either picolinamide or isonicotinamide. Alternatively, but less preferably, the compositions comprise picolinamide and isonicotinamide. In either case, the amount, which is considered individually or collectively, is preferably 0.05 to 8 % by weight of the composition.

At least part of pyridine compound of Formula I or II, either individually or with any other ingredient present in the composition according to the present invention, is not encapsulated. For example, at least 20%, at least 30% and at least 40%, of pyridine compound of formula I or II is not encapsulated.

Preferably, at least 50%, more preferably at least 60%, even more preferably at least 80% and most preferably 100% of pyridine compound of Formula I or II is not encapsulated.

<u>Alkyl substituted resorcinol</u>

Disclosed compositions include an alkyl substituted resorcinol. The alkyl substitution is on one of the ring carbon atoms, preferably C4 and alternatively, but less preferably on C6. Alternatively, the alkyl substitution is on two of the ring carbon atoms, preferably C4 and C6.

In the cases where the alkyl substitution is on one of the ring carbon atoms, preferably C4 and alternatively, but less preferably on C6, it is preferred that the substituted resorcinol is at least one of the following five.

| 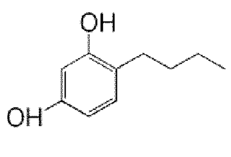 | 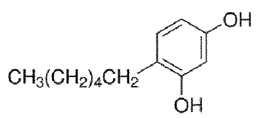 | 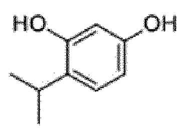 | 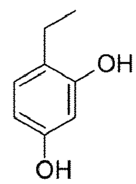 | 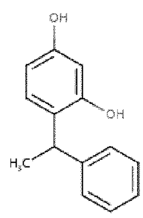 |
|---|---|---|---|---|
| 4-butyl | 4-hexyl | 4-isopropyl | 4-ethyl | 4-phenethyl |

It is further preferred that the substituted resorcinol is 4-butyl resorcinol or 4-hexyl resorcinol or 4-ethyl resorcinol.

Alternatively in cases where the alkyl substitution is on two of the ring carbon atoms, preferably C4 and C6, the substituted resorcinol is represented by Formula III, or Formula IV as follows:

| 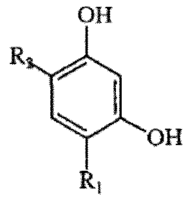 | 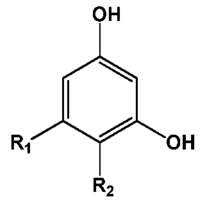 |
|---|---|
| [Formula III] 4,6 di-substitution | [Formula IV] 4,5 di-substitution |

wherein $R^1$ and $R^2$ are alkyl, alkenyl, cycloalkyl, heterocycloalkyl, aryl, heteroaryl, or halo, each of which alkyl, alkenyl, cycloalkyl, heterocycloalkyl, aryl, or heteroaryl is optionally substituted with -OH, $-OR^3$, $-NR^3R^4$, $-C(O)OR^3$, $-C(O)NR^3R^4$, or halo; and $R^3$ and $R^4$ are independently hydrogen, alkyl, alkenyl, cycloalkyl, heterocycloalkyl, cycloalkyl-alkyl, heterocycloalkyl-alkyl, arylalkyl, heteroarylalkyl, aryl, or heteroaryl; or a pharmaceutically acceptable salt thereof. In some variations, $R^1$ is alkyl or halo, and $R^2$ is alkyl or cycloalkyl. In some variations, $R^1$ is alkyl, such as methyl. In some variations, $R^1$ is haloalkyl, perhaloalkyl, fluoroalkyl, or perfluoroalkyl, such as trifluoromethyl. In some variations, $R^1$ is halo, such as fluoro, chloro, bromo, or iodo. In some variations, $R^1$ is fluoro or chloro. In some variations, $R^2$ is alkyl, such as ethyl or hexyl. In some variations, $R^2$ is cycloalkyl, such as cyclohexyl.

In some variations, $R^1$ is $(C_1\text{-}C_6)$-alkyl, $(C_2\text{-}C_6)$-alkenyl, $(C_3\text{-}C_8)$-cycloalkyl, $(C_3\text{-}C_8)$-heterocycloalkyl, $(C_6\text{-}C_{12})$-aryl, $(C_3\text{-}C_{12})$-heteroaryl, or halo, each of which $(C_1\text{-}C_6)$-alkyl, $(C_2\text{-}C_6)$-alkenyl, $(C_3\text{-}C_8)$-cycloalkyl, $(C_3\text{-}C_8)$-heterocycloalkyl, $(C_6\text{-}C_{12})$-aryl, or $(C_3\text{-}C_{12})$-heteroaryl is optionally substituted with one, two, or three substituents selected from the group consisting of -OH, $-OR^3$, $-NR^3R^4$, $-C(O)OR^3$, $-C(O)NR^3R^4$, or halo; and $R^2$ is $(C_1\text{-}C_6)$-alkyl, $(C_2\text{-}C_6)$-alkenyl, $(C_3\text{-}C_8)$-cycloalkyl, $(C_3\text{-}C_8)$-heterocycloalkyl, $(C_6\text{-}C_{12})$-aryl, $(C_3\text{-}C_{12})$-heteroaryl, or halo, each of which $(C_1\text{-}C_6)$-alkyl, $(C_2\text{-}C_6)$-alkenyl, $(C_3\text{-}C_8)$-cycloalkyl, $(C_3\text{-}C_8)$-heterocycloalkyl, $(C_6\text{-}C_{12})$-aryl, or $(C_3\text{-}C_{12})$-heteroaryl is optionally substituted with one, two, or three substituents selected from the group consisting of-OH, $-OR^3$, $-NR^3R^4$, $-C(O)OR^3$, $-C(O)NR^3R^4$, or halo.

In some variations, $R^1$ is $(C_1\text{-}C_6)$-alkyl, $(C_2\text{-}C_6)$-alkenyl, $(C_3\text{-}C_8)$-cycloalkyl, $(C_3\text{-}C_8)$-heterocycloalkyl, $(C_6\text{-}C_{12})$-aryl, $(C_3\text{-}C_{12})$-heteroaryl, or halo, each of which $(C_1\text{-}C_6)$-alkyl, $(C_2\text{-}C_6)$-alkenyl, $(C_3\text{-}C_8)$-cycloalkyl, $(C_3\text{-}C_8)$-heterocycloalkyl, $(C_6\text{-}C_{12})$-aryl, or $(C_3\text{-}C_{12})$-heteroaryl is optionally substituted with one, two, or three substituents selected from the group consisting of -OH, $-OR^3$, $-NR^3R^4$, $-C(O)OR^3$, $-C(O)NR^3R^4$, or halo; and $R^2$ is cycloalkyl, such as $(C_3\text{-}C_8)$-cycloalkyl, such as cyclohexyl.

In some variations, $R^3$ and $R^4$ are independently hydrogen, $(C_1\text{-}C_6)$-alkyl, $(C_2\text{-}C_6)$-alkenyl, $(C_3\text{-}C_8)$-cycloalkyl, $(C_3\text{-}C_8)$-heterocycloalkyl, $(C_3\text{-}C_8)$-cycloalkyl-$(C_1\text{-}C_6)$-alkyl, $(C_3\text{-}C_8)$-heterocycloalkyl-$(C_1\text{-}C_6)$-alkyl, $(C_6\text{-}C_{12})$-aryl-$(C_1\text{-}C_6)$-alkyl, $(C_3\text{-}C_{12})$-heteroaryl-$(C_1\text{-}C_6)$-alkyl, $(C_6\text{-}C_{12})$-aryl, or $(C_3\text{-}C_{12})$-heteroaryl.

In some variations, $R^1$ is $(C_1\text{-}C_6)$-alkyl, $(C_2\text{-}C_6)$-alkenyl, $(C_3\text{-}C_8)$-cycloalkyl, or halo, and $R^2$ is $(C_1\text{-}C_6)$-alkyl, $(C_2\text{-}C_6)$-alkenyl, or $(C_3\text{-}C_8)$-cycloalkyl.

In some variations, $R^1$ is $(C_1\text{-}C_6)$-alkyl or halo, and $R^2$ is $(C_1\text{-}C_6)$-alkyl or $(C_3\text{-}C_8)$-cycloalkyl.

In some variations, $R^1$ is methyl, fluoro, or trifluoromethyl, and $R^2$ is ethyl, hexyl, or cyclohexyl.

In either of formulae, optionally, but less preferably, the hydroxyl groups (the hydrogen on one or both of the OH-groups) may be further substituted by methods known in the art, such as esterification reaction of resorcinol with an acid anhydride. For example, the one or both hydroxyl groups may be esterified with any or a combination of the following acids (or anhydrides thereof): ferulic acid, vanillic acid, sebacic acid, azaleic acid, benzoic acid, caffeic acid, coumaric acid, salicylic acid, cysteine, cystine, lactic acid, and glycolic acid.

It is preferred that the resorcinol has alkyl substitution on the 4-position or the 6-position or at both 4- and 6-positions of the benzene ring. It is preferred that the alkyl group is ethyl, butyl, isopropyl, phenethyl or hexyl group.

It is preferred that the amount of the alkyl substituted resorcinol is 0.01 to 2 % by weight of the composition.

At least part of alkyl substituted resorcinol, either individually or with any other ingredient present in the composition according to the present invention, is not encapsulated. For example, at least 20%, at least 30% and at least 40%, of alkyl substituted resorcinol is not encapsulated.

Preferably, at least 50%, more preferably at least 60%, even more preferably at least 80% and most preferably 100% of alkyl substituted resorcinol is not encapsulated.

It is preferred that the compositions in accordance with the invention comprises one of the following combinations, that of picolinamide and 4-butyl resorcinol, picolinamide and 4-hexyl resorcinol, picolinamide and 4-ethyl resorcinol, isonicotinamide and 4-butyl resorcinol, isonicotinamide and 4-hexyl resorcinol and isonicotinamide and 4-ethyl resorcinol.

Other skin lightening ingredients

The composition of the invention may additionally comprise other skin lightening agents. The skin lightening agent is preferably chosen from a vitamin B3 compound or its derivative e.g. niacin or niacinamide. Other well-known skin lightening agents which may be included are aloe extract, ammonium lactate, azelaic acid, kojic acid, butyl hydroxy anisole, butyl hydroxy toluene, citrate esters, 2, 5 dihydroxybenzoic acid and its derivatives, ellagic acid, gluconic acid, glycolic acid, green tea extract, hydroquinone, 4-hydroxy benzoic acid derivatives, hydroxycaprylic acid, lemon extract, linoleic acid, magnesium ascorbyl phosphate, salicylic acid, or vitamins like vitamin B6, vitamin B12, vitamin C, vitamin A, hydroxycarboxylic acid like lactic acid and their salts e.g. sodium lactate, and mixtures thereof. Vitamin B3 compound or its derivative e.g. niacin or niacinamide are the more preferred additional skin lightening agent as per the invention, most preferred being niacinamide.

Dermatologically Acceptable base

Compositions in accordance with the invention include dermatologically acceptable base, which forms balance of the compositions. The base may act as a diluant, dispersant or carrier for the skin benefit ingredients in the composition, to facilitate their distribution when the composition is applied to the skin.

The base could be aqueous, anhydrous or an emulsion. Preferably, the compositions are aqueous or an emulsion, especially water-in-oil or oil-in-water emulsion, preferentially oil in water emulsion. Water when present will be in amounts which may range from 5 to 99%, preferably from 20 to 70%, optimally between 40 and 70% by weight.

Besides water, relatively volatile solvents may also serve as carriers within compositions of the present invention. Most preferred are monohydric C1-C3 alcohols These include ethyl alcohol, methyl alcohol and isopropyl alcohol. The amount of monohydric alcohol may range from 1 to 70%, preferably from 10 to 50%, optimally between 15 to 40% by weight.

Emollient materials may also serve as cosmetically acceptable carriers. These may be in the form of silicone oils and synthetic esters. Amounts of the emollients may range anywhere from 0.1 to 50%, preferably between 1 and 20% by weight.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, preferably from 4 to 5, silicon atoms. Linear volatile silicones generally have viscosities less than about 5 centistokes at 25 °C while cyclic materials typically have viscosities of less than about 10 centistokes. Nonvolatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 to about 25 million centistokes at 25°C. Among the preferred non-volatile emollients useful in the present compositions are the polydimethyl siloxanes having viscosities from about 10 to about 400 centistokes at 25 °C.

Among the ester emollients are:

(i) alkenyl or alkyl esters of fatty acids having 10 to 20 carbon atoms. Examples thereof include isoarachidyl neopentanoate, isononyl isonanonoate, oleyl myristate, oleyl stearate, and oleyl oleate;

(ii) ether-esters such as fatty acid esters of ethoxylated fatty alcohols;

(iii) polyhydric alcohol esters such as ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.

(iv) wax esters such as beeswax, spermaceti, myristyl myristate, stearyl stearate and arachidyl behenate; and,

(v) sterol esters, of which cholesterol fatty acid esters are examples.

Fatty acids having from 10 to 30 carbon atoms may also be included as cosmetically acceptable carriers for compositions of this invention. Illustrative of this category are pelargonic, lauric, myristic, palmitic, stearic, isostearic, hydroxystearic, oleic, linoleic, ricinoleic, arachidic, behenic and erucic acids.

Humectants of the polyhydric alcohol-type may also be employed as dermatologically acceptable base in compositions of this invention. The humectant aids in increasing the effectiveness of the emollient, reduces scaling, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably propylene glycol or sodium hyaluronate. The amount of humectant may range anywhere from 0.5 to 30%, preferably between 1 and 15% by weight of the composition.

Thickeners may also be utilized as part of the cosmetically dermatologically acceptable base of compositions according to the present invention. Typical thickeners include crosslinked acrylates (e.g. Carbopol® 982), hydrophobically-modified acrylates (e.g. Carbopol® 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.0001 to 5%, usually from 0.001 to 1%, optimally from 0.01 to 0.5% by weight.

Collectively the water, solvents, silicones, esters, fatty acids, humectants and/or thickeners will constitute the dermatologically acceptable base in amounts from 1 to 99.9%, preferably from 80 to 99% by weight.

An oil or oily material may be present, together with an emulsifier to provide either a water-in-oil emulsion or an oil-in-water emulsion, depending largely on the average hydrophilic-lipophilic balance (HLB) of the emulsifier employed.

Surfactants may also be present in compositions of the present invention. Total concentration of the surfactant will range from 0.1 to 40%, preferably from 1 to 20%, optimally from 1 to 5% by weight of the composition. The surfactant may be selected from the group consisting of anionic, nonionic, cationic and amphoteric actives. Particularly preferred nonionic surfactants are those with a $C_{10}$-$C_{20}$ fatty alcohol or acid hydrophobe condensed with from 2 to 100 moles of ethylene oxide or propylene oxide per mole of hydrophobe; $C_2$ to $C_{10}$ alkyl phenols condensed with from 2 to 20 moles of alkylene oxide; mono- and di-fatty acid esters of ethylene glycol; fatty acid monoglyceride; sorbitan, mono- and di-$C_8$ to $C_{20}$ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, $C_8$ to $C_{20}$ acyl isethionates, acyl glutamates, $C_8$ to $C_{20}$ alkyl ether phosphates and combinations thereof.

It is preferred that dermatologically acceptable bases are such as to have a product in preferably a cream format. A preferred format is a vanishing cream base. Vanishing cream base comprises 1 to 25%, preferably 5 to 25%, more preferably 5 to 20% by weight fatty acid. This base also comprises 0.1 to 10%, more preferably 0.1 to 3% by weight soap. Preferably, the base comprises both fatty acid and soap. $C_{12}$ to $C_{20}$ fatty acids are especially preferred in vanishing cream bases, further more preferred being $C_{12}$ to $C_{18}$ fatty acids. In creams, the fatty acid is preferably substantially a mixture of stearic acid and palmitic acid. Soaps in the vanishing cream base include alkali metal salt of fatty acids, like sodium or potassium salts. The soap is preferably the potassium salt of the fatty acid mixture. The fatty acid in vanishing cream base is often prepared using hysteric acid which is substantially (generally about 90 to 95%) a mixture of 45-47% stearic acid and 53-55% palmitic acid. Thus, inclusion of hysteric acid and its soap to prepare the vanishing cream base is within the scope of the present invention. It is particularly preferred that the composition comprises higher than 7%, preferably higher than 10%, more preferably higher than 12% by weight fatty acid. The cosmetically acceptable base is usually from 10 to 99.9%, preferably from 50 to 99% by weight of the composition. The cosmetically acceptable base preferably includes water. Water is preferably included in 35 to 90%, more preferably 50 to 85%, further more preferably 50 to 80% by weight of the composition. Generally, the vanishing cream base in personal care compositions is prepared by taking a desired amount of total fatty matter and mixing with potassium hydroxide in desired amounts. The soap is usually formed in-situ during the mixing.

Further Optional Ingredients

Other adjunct or functional ingredients are preferably also incorporated into the cosmetic compositions of the invention. These ingredients may include coloring agents, pigments, opacifiers, and perfumes. Amounts of these other adjunct minor components may range anywhere from 0.001% up to 20% by weight of the composition.

Sunscreens

For use as sunscreen, metal oxides may be used alone or in mixture and/or in combination with organic sunscreens. Examples of organic sunscreens include but are not limited those set forth below.

The amount of the organic sunscreens in the compositions is preferably in the range of about 0.1 wt % to about 10 wt %, more preferably about 1 wt % to 5 wt %. Preferred organic sunscreens are Parsol® MCX and Parsol® 1789, due to their effectiveness and commercial availability.

Perfumes may be used in the composition of this invention. Illustrative non-limiting examples of the types of perfumes that may be used include those comprising terpenes and terpene derivatives like those described in Bauer, K., et al., Common Fragrance and Flavor Materials VCH Publishers (1990)).

Illustrative yet non-limiting examples of the types of fragrances that may be used in this invention include myrcene, dihydromyrenol, citral, tagetone, cis- geranic acid, citronellic acid, or cis-geranic acid nitrile, mixtures thereof or the like. Preferably, the amount of fragrance employed in the compositions of this invention is in the range from about 0.0% to about 10%, more preferably, about 0.00001% to about 5 wt %, most preferably, about 0.0001% to about 2%.

Various types of optional additional active ingredients may be used in the skin care compositions of the present invention. Actives are defined as skin benefit agents other than emollients and other than ingredients that merely improve the physical characteristics of the composition. Although not limited to this category, general examples include talcs and silicas, as well as alpha- hydroxy acids, beta-hydroxy acids, poly-hydroxy acids, peroxides and zinc salts.

Beta-hydroxy acids include salicylic acid, for example. Zinc pyrithione is an example of the zinc salts useful in the compositions of the present invention.

Many skin care compositions, especially those containing water, should be protected against the growth of potentially harmful microorganisms. Anti-microbial compounds, such as triclosan, and preservatives are, therefore, typically necessary. Suitable preservatives include alkyl esters of p- hydroxybenzoic acid, hydantoin derivatives, propionate salts, and a variety of quaternary ammonium compounds. Particularly preferred preservatives of this invention are methyl paraben, propyl paraben, phenoxyethanol and benzyl alcohol. Preservatives will usually be employed in amounts ranging from about 0.1% to 2% by weight of the composition.

Method of using the composition

The method according to the invention is intended primarily for using as a personal care product for topical application to human skin, as well as to lighten the skin, to reduce the degree of pigmentation in the skin, or to even the skin tone.

In use, a small quantity of the composition, for example from 1 to 5 ml, is applied to exposed areas of the skin, from a suitable container or applicator and, if necessary, it is then spread over and/or rubbed into the skin using the hand or fingers or a suitable device.

Product form and packaging

Compositions in accordance with the invention are preferably a lotion having viscosity of from 4,000 to 10,000 mPas, a fluid cream having a viscosity of from 10,000 to 20, 000 mPas, or a cream having a viscosity of from 20,000 to 100,000 mPas or above. The composition can be packaged in a suitable container to suit its viscosity and intended use by the consumer. For example, a lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar. When the composition is a solid or semi-solid stick, it may be packaged in a suitable container for manually or mechanically pushing out or extruding the composition.

Other aspects of the invention:

In accordance with a second aspect is disclosed a method for enhancing TDD of an alkyl substituted resorcinol, comprising the steps of:

(i) providing a cosmetic composition in accordance with the first aspect; and,
(ii) applying an effective amount of said composition to skin.

It is preferred that the method is of non-therapeutic nature.

In accordance with a third aspect is disclosed use of a pyridine compound of Formula I or II for enhancing TDD of an alkyl substituted resorcinol. It is preferred that the use is for non-therapeutic purposes, e.g., for cosmetic applications.

In accordance with a fourth aspect is disclosed use of a pyridine compound of Formula I or II in the preparation of a cosmetic composition for enhancing TDD of an alkyl substituted resorcinol. In this case also it is preferred that the use is for non-therapeutic purposes, e.g., for cosmetic applications.

In accordance with a fifth aspect is disclosed a composition for use to enhance TDD of an alkyl substituted resorcinol, comprising:

(i) at least one pyridine compound of Formula I or II:

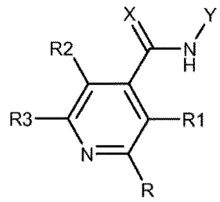

Formula I

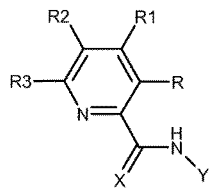

Formula II

where, in said Formula I and in said Formula II, X is S or O; R, $R_1$, $R_2$, $R_3$ are either -H, -SH, -OH or a $C_{1-18}$ saturated or unsaturated, linear, branched or cyclic hydrocarbon group; Y is -H, -SH or $C_{1-18}$ saturated or unsaturated, linear, branched or cyclic hydrocarbon group;
(ii) an alkyl substituted resorcinol; and,
(iii) a dermatologically acceptable base.

In accordance with a sixth aspect is disclosed a method of lightening skin comprising a step of applying thereon a composition in accordance with the first aspect.

The invention accordingly also provides a closed container containing a cosmetically acceptable composition as herein defined.

The following examples are by way of example, not by way of limitation, of the principles of the present invention, to illustrate the mode of carrying out the invention.

**Examples**

Example 1: TDD using Franz diffusion cells

A cosmetic composition (lotion) containing picolinamide and 4-hexyl resorcinol was prepared using the ingredients shown in Table 1. Another lotion containing isonicotinamide and 4-hexyl resorcinol was also prepared. In addition to these two compositions, a third composition (Comparative/Outside the scope of present invention) was prepared. This composition was devoid of picolinamide as well as isonicotinamide.

Table 1: Compositions and their Respective % TDD

| Ingredients/wt% | Composition No. | | |
|---|---|---|---|
| | **1** | **2** | **3** |
| Potassium Stearate | 3.0 | 3.0 | 3.0 |
| Stearic acid | 14.0 | 14.0 | 14.0 |
| Micronised Titanum Dioxide | 0.6 | 0.6 | 0.6 |
| Isopropyl myristate | 0.7 | 0.7 | 0.7 |
| Parsol® MCX | 0.7 | 0.7 | 0.7 |
| Parsol® 1789 | 0.4 | 0.4 | 0.4 |
| 4-hexyl resorcinol | 1.0 | 1.0 | 1.0 |
| Picolinamide | - | 3.0 | - |
| Isonicotinamide | - | - | 3.0 |
| Water and other minors to | 100.0 | 100.0 | 100.0 |
| % penetration in TDD | 22.8 | 26.5 | 23.2 |

All the compositions of Table 1 were tested according to the procedure described below.

The Diffusion Experiment

On the day of experiment, skin samples were thawed in a sequential manner from -20 °C to 0 °C and then to

4 °C and finally at room temperature. Hair were removed with an electric shaver and the full thickness pig skin (-1.3 mm) was cut into circular disc of diameter -4.5 cm. The discs were mounted on Franz diffusion cells having diameter 2.498 cm. The area therefore available for diffusion was 4.91 $cm^2$. With every set of experiment, caffeine was run in duplicate for testing of skin integrity. This is required for the acceptance of data for permeation of active molecules. Jacketed Franz diffusion cell manufactured by Perme Gear Inc, USA, with appropriate dimensions (ID: 2.5 cm, OD: 4.4 cm, Receptor Volume: 25 ml, Diffusion area: 4.91 $cm^2$) were used for this experiment. A one % solution of Tween® 80 in water (Phosphate Buffer Saline, PBS) was taken as the receptor solution in Franz diffusion cell. The internal temperature was maintained at 32 °C by water jacket. After mounting the skin with epidermis on the diffusion cell, it was equilibrated to 32 °C for 1 to 2 hours prior to application of the concerned formulation. About 100 mg formulation was applied on the pig skin with help of an applicator, which, along with formulation, was weighed before and after the application to calculate the actual amount of formulation applied. At the end of 24 hours, the receptor solution was drawn from receptor compartment, donor side was washed with 5 ml aliquots total of 10 to 15 ml of 1% Tween® 80 in aqueous solution. The skin was cut to small pieces with a scalpel and homogenized with -15 ml of 1% Tween® 80 in water. Donor and skin extracts were sonicated for 2 to 3 hours to ensure total extraction of active molecule in the solution.

Determination of Skin Integrity

To test the integrity of an isolated skin sample, 0.5 ml of a 1% Caffeine solution in PBS was applied on an area of 1.77 $cm^2$ of the skin to be tested. The receptor fluid was collected at the end of 24 hours and 48 hours for concentration of Caffeine. Care was taken to ensure that the amount of permeated Caffeine did not exceed 0.2 % of applied dose.

Preparation of Formulation Sample Solution

100 mg of sample (All formulations individually) was weighed in a volumetric flask. A 1% solution of Tween® 80 in distilled water (about 10 ml) was added to it and sonicated for 10 minutes. This solution was filtered through 0.2 μm syringe filter to give final concentration of 10 mg/ml.

Sample Analysis

Receptor solution, skin homogenate and skin wash (Donor) samples were analysed to determine the concentration of the resorcinol in each case using an HPLC method. Percentage recovery of the active was calculated for each compartment as follows:

$$X\ (g/g) = ((Ci \times V \times 100)/m)$$

where, X (g/g): the content of active in sample , the unit is g/g
m = sample weight, the unit is gram (g)
Ci = the concentration of active in same solution, the unit is microgram per milliliter (μg/ml) and V = the volume of sample solution, the unit is milliliter (ml).

The data in Table 1 indicates clearly how picolinamide and isonicotinamide effect the TDD of the resorcinol in the case of Compositions 2 and 3. The Comparative Composition 1 did not show significantly appreciable effect, but Composition 2 containing 4-hexyl resorcinol and picolinamide and Composition 3 containing 4-hexyl resorcinol and isonicotinamide showed enhanced TDD.

Example 2: In-vitro experiments on inhibition of melanin using Picolinamide

In-vitro experiments were conducted using the comparative test samples and test samples according to the present invention as provided below. The objective of this experiment was to ascertain the extent of skin lightening brought about by the combination of ingredients in accordance with this invention.

All samples were analysed to determine the extent of their ability to bring about skin lightening by checking the cell viability and melanin content assay.

Set up of cultures involved the following steps:

(i) 5X10$^4$ HeMnDP per well (single wells/treatment) in 24-well Nunc plates on Day-0 were seeded to establish a melanocyte monoculture system.
(ii) The volume of media per well was limited to 1 ml of Melanocyte Growth Media (MGM)
(iii) 24 hours later, fresh media containing actives was added again. At this stage also, 1 ml of MGM was used.
(iv) Plates were incubated for 72 hours at 37 °C in a $CO_2$ incubator.
(v) At the end, the plates were sent for Calcein assay, described hereinafter, followed by melanin content estimation.

Calcein-AM viability assay

(i) The spent media was removed from each well of a 24-well plate and rinsed once with 400 μl of 1xPBS-Ca-Mg (see preparation below), including wells without cells (Control wells).
(ii) One μM Calcein-AM (working stock) in 1xPBS-Ca-Mg from 1 mM stock (e.g. 10 μl of 1 mM Calcein stock in 100 % DMSO in 10 ml of 1xPBS-Ca-Mg) was prepared. About 400 μl of Calcein-AM working stock solution was added to each well, including the controls cells.
(iii) The plates were covered with aluminum foil and were incubated for 30 minutes in a $CO_2$ incubator.
(iv) In the meantime, fluorescence was measured (excitation at 490 nm and emission at 520 nm) using TECAN® instrument after 30 minutes of dye incubation.
(v) After reading the plates, the buffer containing Calcein was removed and melanin content assay was then carried out.

Preparation of PBS-Ca-Mg

20X PBS-Ca-Mg stock buffers (for 1 litre solution)

Solution A:

| | |
|---|---|
| 348 mM $Na_2HPO_4$ (anhydrous) | 49.4 g |
| 70 mM $NaH_2PO_4$ (dihydrate) | 10.92 g |

Solution B:

| | |
|---|---|
| 18 mM $CaCl_2$ | 2.6g |
| 70 mM KCl | 5.22 g |
| 18 mM $MgCl_2$ (hexahydrate) | 3.66 g |
| 2740 mM NaCl | 160.3 g |

50 mL of each of Solution A and B were prepared in autoclaved distilled water and stored at room temperature.
5 mL each of solutions A and B are taken and 90 ml of water is added to it, to prepare a working stock solution which was filtered before use.

Preparation of 1 mM Calcein-AM stock

(i) 500 μl of 100% DMSO was added to 1 mg Calcein AM (Fluka/Sigma Aldrich, CAS No 148504-34-1).

(ii) The ingredients were mixed and another 500 μl of 100% DMSO was added to it.

(iii) The mixture was further well mixed, taken in 20 μl aliquot, which was covered with Al foil and stored at 20 °C.

Melanin Content assay

Preparation of MCA reagent (10 % DMSO in 1N NaOH)

Table 2

| Volume of MCA reagent/ml | Volume of DMSO (on 100% basis)/ml | Volume of 10 N NaOH/ml | Volume of water/ml |
|---|---|---|---|
| 10 | 1 | 1 | 8 |

(i) After recordal of the reading for Calcein-AM, the buffer containing Calcein-AM was removed from all wells.

(ii) Thereafter, 150 μL MCA reagent was added to each well.

(iii) The plates were incubated for 1 hour at 60 °C with gentle shaking.

(iv) 120 μL of solution was transferred from each well into a 384-well plate.

(v) The absorbance was measured at 405 nm.

(vi) For non-cytotoxic concentrations (based on Calcein cut-off of +/- 15 % of control), the average MCA was calculated and reported as MCA, as the percentage of the control sample. The % inhibition was then calculated for each case.

Higher values of % inhibition indicate an active ingredient's potential to serve as a skin lightening agent.

Table 3

| Samples | Treatment | Inhibition in melanin Synthesis | Standard Deviation |
|---|---|---|---|
| Ex - A | Untreated | 0 | -NA- |
| Ex - B | 4-hexyl Resorcinol 0.192 μg | 3 | 0.02 |
| Ex - C | 4-hexyl Resorcinol 1.92 μg | 31 | 0.008 |
| Ex - D | picolinamide 122 μg | 1 | 0.01 |
| Ex - E | picolinamide 1220 μg | 27 | 0.02 |
| Ex - 1 | 4-hexyl Resorcinol 0.192 μg +picolinamide 122 μg | 13 | 0.029 |
| Ex - 2 | 4-hexyl Resorcinol 0.192 μg +picolinamide 1220 μg | 34 | 0.02 |
| Ex - 3 | 4-hexyl Resorcinol 1.92 μg +picolinamide 122μg | 42 | 0.03 |
| Ex - 4 | 4-hexyl Resorcinol 1.92 μg +picolinamide 1220 μg | 48 | 0.007 |

The data in Table 3 indicates that samples stimulating the conditions of compositions in accordance with the invention (Example 1, 2, 3 4) caused significantly more inhibition in melanin synthesis as compared to the Control (A, B, C, D and E).

Example 3: In-vitro experiments on the inhibition of melanin using Isonicotinamide

The tests of Example 2 were repeated with isonicotinamide instead of picolinamide.
The information is summarised in Table 4.

Table 4

| Samples | Treatment | Inhibition in melanin synthesis | SD |
|---|---|---|---|
| Ex - F | Untreated | 0 | |

(continued)

| Samples | Treatment | Inhibition in melanin synthesis | SD |
|---|---|---|---|
| Ex - G | 4-hexyl Resorcinol 0.192 μg | 3 | 0.02 |
| Ex - H | 4-hexyl Resorcinol 1.92 μg | 31 | 0.008 |
| Ex - I | Isonicotinamide 122 μg | -16 | 0.01 |
| Ex - J | Isonicotinamide 1220 μg | 10 | 0.02 |
| Ex - 5 | 4-hexyl Resorcinol 0.192 μg + Isonicotinamide 122 μg | 8 | 0.1 |
| Ex - 6 | 4-hexyl Resorcinol 0.192 μg + Isonicotinamide 1220 μg | 21 | 0.02 |
| Ex - 7 | 4-hexyl Resorcinol 1.92 μg + Isonicotinamide 122 μg | 41 | 0.05 |
| Ex - 8 | 4-hexyl Resorcinol 1.92 μg + Isonicotinamide 1220 μg | 52 | 0.04 |

The data in Table 4 also indicates that samples stimulating the conditions of compositions in accordance with the invention (Example 5, 6, 7 and 8) brought about significantly more inhibition in melanin synthesis as compared to the corresponding Control samples.

## Claims

**1.** A cosmetic composition comprising:

(i) at least one pyridine compound of Formula I or II:

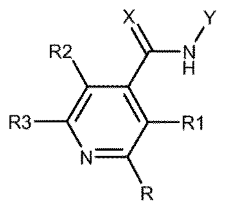

Formula I

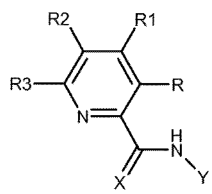

Formula II

where, in said Formula I and in said Formula II, X is S or O; R, $R_1$, $R_2$, $R_3$ are either -H, -SH, -OH or a $C_{1-18}$ saturated or unsaturated linear, branched or cyclic hydrocarbon group; Y is -H, -SH or $C_{1-18}$ saturated or unsaturated, linear, branched or cyclic hydrocarbon group;
(ii) an alkyl substituted resorcinol; and,
(iii) a dermatologically acceptable base.

**2.** A composition as claimed in claim 1 wherein ratio of the amount of said alkyl substituted resorcinol to said pyridine compound of Formula I or Formula II is from 1:0.25 to 1:10 parts by weight.

**3.** A composition as claimed in claim 1 or 2 wherein amount of said pyridine compound of Formula I or II is 0.05 to 8 % by weight of the composition.

**4.** A composition as claimed in any preceding claim 1 to 3 wherein amount of said alkyl substituted resorcinol is 0.01 to 2 % by weight of the composition.

**5.** A cosmetic composition as claimed in any preceding claim 1 to 4 wherein the pyridine compound is at least one of isonicotinamide or picolinamide.

**6.** A cosmetic composition as claimed in any preceding claim 1 to 5 wherein said resorcinol has alkyl substitution on the 4-position or the 6-position or at both 4-and 6- positions of the benzene ring.

**7.** A composition as claimed in claim 6 wherein said alkyl group is ethyl, butyl, isopropyl, phenethyl or hexyl group.

8. A composition as claimed in claim 7 comprising picolinamide and 4-hexyl resorcinol.

9. A composition as claimed in claim 7 comprising isonicotinamide and 4-hexyl resorcinol.

10. A composition as claimed in claim 7 comprising isonicotinamide and 4-isopropyl resorcinol.

11. A composition as claimed in claim 7 comprising picolinamide and 4-isopropyl resorcinol.

12. A method for enhancing transdermal delivery of an alkyl substituted resorcinol comprising the steps of:

(i) providing a cosmetic composition as claimed in claim 1; and,
(ii) applying an effective amount of said composition to skin.

13. Use of a pyridine compound of Formula I or II for enhancing transdermal delivery of an alkyl substituted resorcinol.

14. Use of a pyridine compound of Formula I or II in the preparation of a cosmetic composition for enhancing transdermal delivery of an alkyl substituted resorcinol.

15. A composition for use to enhance transdermal delivery of an alkyl substituted resorcinol, comprising:

(i) at least one pyridine compound of Formula I or II:

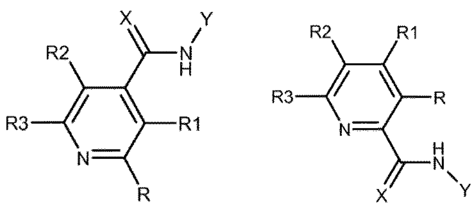

Formula I Formula II

where, in said Formula I and in said Formula II, X is S or O; R, $R_1$, $R_2$, $R_3$ are either -H, -SH, -OH or a $C_{1-18}$ saturated or unsaturated, linear, branched or cyclic hydrocarbon group; Y is -H, -SH or $C_{1-18}$ saturated or unsaturated, linear, branched or cyclic hydrocarbon group;
(ii) an alkyl substituted resorcinol; and,
a dermatologically acceptable base.

**Patentansprüche**

1. Kosmetische Zusammensetzung, umfassend:

(i) mindestens eine Pyridin-Verbindung der Formel I oder II:

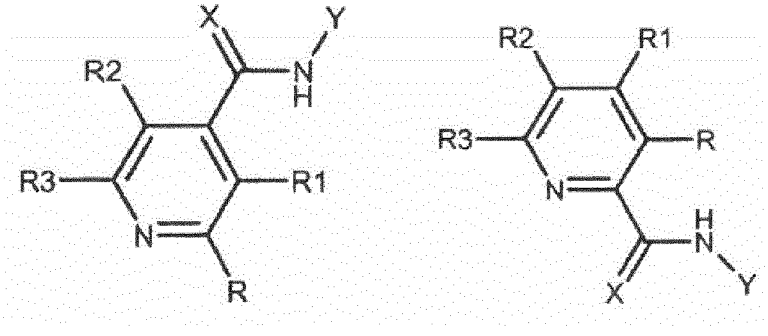

Formel I Formel II

wobei in der Formel I und in der Formel II X S oder O ist; R, $R_1$, $R_2$, $R_3$ entweder -H, -SH, -OH oder eine gesättigte oder ungesättigte lineare, verzweigte oder cyclische $C_1$-$C_{18}$-Kohlenwasserstoffgruppe sind; Y -H, -SH oder eine gesättigte oder ungesättigte, lineare, verzweigte oder cyclische $C_1$-$C_{18}$-Kohlenwasserstoffgruppe ist;
(ii) ein Alkyl-substituiertes Resorcin; und
(iii) einen dermatologisch verträglichen Grundbestandteil.

**2.** Zusammensetzung, wie im Anspruch 1 beansprucht, wobei das Verhältnis der Menge des Alkyl-substituierten Resorcins zu der Pyridin-Verbindung der Formel I oder Formel II von 1:0,25 bis 1:10 Gewichtsteilen beträgt.

**3.** Zusammensetzung, wie im Anspruch 1 oder 2 beansprucht, wobei die Menge der Pyridin-Verbindung der Formel I oder II 0,05 bis 8 Gewichts-% der Zusammensetzung beträgt.

**4.** Zusammensetzung, wie in irgendeinem vorhergehenden Anspruch 1 bis 3 beansprucht, wobei die Menge des Alkyl-substituierten Resorcins 0,01 bis 2 Gewichts-% der Zusammensetzung beträgt.

**5.** Kosmetische Zusammensetzung, wie in irgendeinem vorhergehenden Anspruch 1 bis 4 beansprucht, wobei die Pyridin-Verbindung mindestens eines aus Isonicotinamid oder Picolinamid ist.

**6.** Kosmetische Zusammensetzung, wie in irgendeinem vorhergehenden Anspruch 1 bis 5 beansprucht, wobei das Resorcin eine Alkyl-Substitution in der 4-Stellung oder der 6-Stellung oder in beiden 4- und 6-Stellungen des Benzolrings aufweist.

**7.** Zusammensetzung, wie im Anspruch 6 beansprucht, wobei die Alkylgruppe eine Ethyl-, Butyl-, Isopropyl-, Phenethyl- oder Hexyl-Gruppe ist.

**8.** Zusammensetzung, wie im Anspruch 7 beansprucht, umfassend Picolinamid und 4-Hexylresorcin.

**9.** Zusammensetzung, wie im Anspruch 7 beansprucht, umfassend Isonicotinamid und 4-Hexylresorcin.

**10.** Zusammensetzung, wie im Anspruch 7 beansprucht, umfassend Isonicotinamid und 4-Isopropylresorcin.

**11.** Zusammensetzung, wie im Anspruch 7 beansprucht, umfassend Picolinamid und 4-Isopropylresorcin.

**12.** Verfahren zur Verbesserung der transdermalen Abgabe eines Alkyl-substituierten Resorcins, umfassend die Schritte:

(i) Bereitstellen einer kosmetischen Zusammensetzung, wie im Anspruch 1 beansprucht; und
(ii) Auftragen einer wirksamen Menge der Zusammensetzung auf die Haut.

**13.** Verwendung einer Pyridin-Verbindung der Formel I oder II zum Verbessern der transdermalen Abgabe eines Alkyl-substituierten Resorcins.

**14.** Verwendung einer Pyridin-Verbindung der Formel I oder II zur Herstellung einer kosmetischen Zusammensetzung zum Verbessern der transdermalen Abgabe eines Alkyl-substituierten Resorcins.

**15.** Zusammensetzung zur Verwendung zum Verbessern der transdermalen Abgabe eines Alkyl-substituierten Resorcins, umfassend:

(i) mindestens eine Pyridin-Verbindung der Formel I oder II:

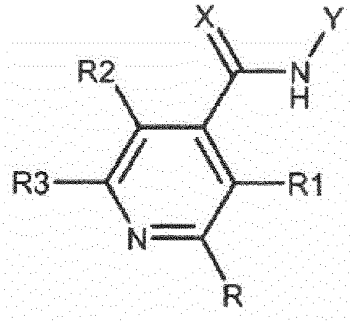

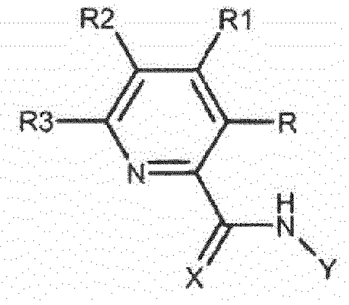

Formel I Formel II

wobei in der Formel I und in der Formel II X S oder O ist; R, $R_1$, $R_2$, $R_3$ entweder -H, -SH, -OH oder eine gesättigte oder ungesättigte lineare, verzweigte oder cyclische $C_1$-$C_{18}$-Kohlenwasserstoffgruppe sind; Y -H, -SH oder eine gesättigte oder ungesättigte, lineare, verzweigte oder cyclische $C_1$-$C_{18}$-Kohlenwasserstoffgruppe ist;
(ii) ein Alkyl-substituiertes Resorcin; und einen dermatologisch verträglichen Grundbestandteil.

## Revendications

**1.** Composition cosmétique comprenant :

(i) au moins un composé de pyridine de formule I ou II :

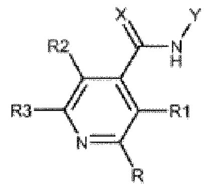

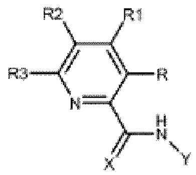

Formule I Formule II

où dans ladite formule I et dans ladite formule II, X est S ou O ; R, $R_1$, $R_2$, $R_3$ sont soit -H, -SH, -OH soit un groupe hydrocarboné linéaire, ramifié ou cyclique saturé ou insaturé en $C_{1-18}$; Y est -H, -SH ou un groupe hydrocarboné linéaire, ramifié ou cyclique saturé ou insaturé en $C_{1-18}$ ;
(ii) un résorcinol substitué par un groupe alkyle ; et
(iii) une base dermatologiquement acceptable.

**2.** Composition selon la revendication 1, dans laquelle le rapport de la quantité dudit résorcinol substitué par un groupe alkyle audit composé de pyridine de formule I ou formule II est de 1:0,25 à 1:10 parties en masse.

**3.** Composition selon la revendication 1 ou 2, dans laquelle la quantité dudit composé de pyridine de formule I ou II est de 0,05 à 8 % en masse de la composition.

**4.** Composition selon l'une quelconque des revendications 1 à 3 précédentes, dans laquelle la quantité dudit résorcinol substitué par un groupe alkyle est de 0,01 à 2 % en masse de la composition.

**5.** Composition cosmétique selon l'une quelconque des revendications 1 à 4 précédentes, dans laquelle le composé de pyridine est au moins un d'isonicotinamide ou picolinamide.

**6.** Composition cosmétique selon l'une quelconque des revendications 1 à 5, dans laquelle ledit résorcinol présente une substitution alkyle sur la position 4 ou la position 6 ou à la fois aux positions 4 et 6 du noyau benzène.

**7.** Composition selon la revendication 6, dans laquelle ledit groupe alkyle est un groupe éthyle, butyle, isopropyle,

phénéthyle ou hexyle.

**8.** Composition selon la revendication 7 comprenant du picolinamide et 4-hexylrésorcinol.

**9.** Composition selon la revendication 7 comprenant de l'isonicotinamide et 4-hexylrésorcinol.

**10.** Composition selon la revendication 7 comprenant de l'isonicotinamide et 4-isopropylrésorcinol.

**11.** Composition selon la revendication 7 comprenant du picolinamide et 4-isopropylrésorcinol.

**12.** Procédé de promotion de libération transdermique d'un résorcinol substitué par un groupe alkyle comprenant les étapes de :

(i) fourniture d'une composition cosmétique selon la revendication 1 ; et,
(ii) application d'une quantité efficace de ladite composition sur la peau.

**13.** Utilisation d'un composé de pyridine de formule I ou II pour promouvoir la libération transdermique d'un résorcinol substitué par un groupe alkyle.

**14.** Utilisation d'un composé de pyridine de formule I ou II dans la préparation d'une composition cosmétique pour promouvoir la libération transdermique d'un résorcinol substitué par un groupe alkyle.

**15.** Composition pour une utilisation pour promouvoir la libération transdermique d'un résorcinol substitué par un groupe alkyle, comprenant :

(i) au moins un composé de pyridine de formule I ou II :

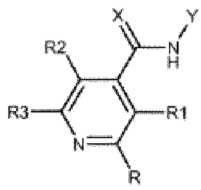

Formule I

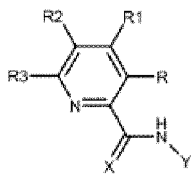

Formule II

où dans ladite formule I et dans ladite formule II, X est S ou O ; R, $R_1$, $R_2$, $R_3$ sont soit -H, -SH, -OH soit un groupe hydrocarboné linéaire, ramifié ou cyclique saturé ou insaturé en $C_{1-18}$ ; Y est -H, -SH ou un groupe hydrocarboné linéaire, ramifié ou cyclique saturé ou insaturé en $C_{1-18}$ ;
(ii) un résorcinol substitué par un groupe alkyle ; et
une base dermatologiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 341664 A1
- WO 9915148 A1
- US 4959393 A
- US 6132740 A
- US 6504037 B
- US 20080131382
- JP 2001010925 A
- JP 2000327557 A
- US 5399785 A
- US 20040042983
- US 2014256830 A
- US 2013281507 A
- EP 0623339 A1
- WO 13190483 A1
- US 2002120225 A
- US 5411741 A
- WO 2013030794 A2
- WO 2015059001 A


### Non-patent literature cited in the description


- *Tropical Journal of Pharmaceutical Research,* April 2009, vol. 8 (2), 173-179
- **BAUER, K. et al.** Common Fragrance and Flavor Materials. VCH Publishers, 1990
- *CHEMICAL ABSTRACTS,* 148504-34-1